Europäisches Patentamt

(19) European Patent Office

Office européen des brevets

(11) Publication number: **0 001 156**
**B1**

(12) **EUROPEAN PATENT SPECIFICATION**

(45) Date of publication of patent specification: **11.05.83**

(51) Int. Cl.³: **A 61 N 1/36, G 08 C 25/00**

(21) Application number: **78300239.7**

(22) Date of filing: **03.08.78**

(54) **Programmable, implantable body function control apparatus and method for reprogramming said apparatus.**

(30) Priority: **19.08.77 GB 3491177**
**19.06.78 US 917130**

(43) Date of publication of application:
**21.03.79 Bulletin 79/6**

(45) Publication of the grant of the patent:
**11.05.83 Bulletin 83/19**

(84) Designated Contracting States:
**DE FR GB NL SE**

(56) References cited:
**DE - A - 2 030 474**
**LU - A - 76 880**
**US - A - 3 805 796**
**US - A - 3 906 348**

(73) Proprietor: **BIOTRONIK Mess- und Therapiegeräte GmbH & Co Ingenieurbüro Berlin Sieversufer 8**
**D-1000 Berlin 47 (DE)**

(72) Inventor: **Keller, John Walter**
**8600 S.W. 54th Avenue**
**Miami Florida (US)**
Inventor: **Digby, Dennis**
**2409 Gunflint Trail**
**Brooklyn Park Minnesota 55444 (US)**
Inventor: **Coombes, Alan**
**8103 28th Avenue North**
**New Hope Minnesota 55427 (US)**

(74) Representative: **Christiansen, Henning, Dipl.-Ing.**
**Unter den Eichen 108a**
**D-1000 Berlin 45 (DE)**

Courier Press, Leamington Spa, England.

# Programmable, implantable body function control apparatus and method for reprogramming said apparatus

## Technical field

This invention relates to implantable body function control apparatus and particularly, but no exclusively, to body tissue stimulating devices such as cardiac pacemakers.

## Background art

Pacemakers for generating artificial stimulating pulses for the heart, and which may be implanted in the body, are well known. Originally the electrical circuitry for such pacemakers was of analog design, but in recent years digital circuitry has been also employed. A digital approach to pacemakers has led to the evolution of programmable pacemakers—pacemakers having parameters such as pulse rates which are adjustable (programmable) once the pacemaker has been implanted. Programmable pacemakers are described in, for instance, British Specifications 1,385,954 and 1,398,875. Such pacemakers have circuitry to detect and decode signals transmitted outside the body and alter the program accordingly. In British Specification 1,385,954 (claiming priority based on USSN 141,694, in turn a parent of U.S.P.N. 3,805,796 to Tenz) the programming is accomplished by means of a magnetic field which is sensed by a magnetic reed switch; the opening and closing of the switch provides programming pulses to a program store. In British Specification 1,398,875 (based on U.S.P.N. 3,833,005 to Wingrove) the programming is by means of radio frequency transmission and reception.

It is clearly of paramount importance that the stored program is such equipment is only altered when desired and not, for example, in response to electrical noise generated in proximity to the pacemaker. If the pacemaker is arranged to receive and decode radio frequency signals for changing the stored program then clearly precautions must be taken to avoid any undesired radio frequency transmissions from altering the program.

From LU—A—76880 ("Walters") a programmable pacemaker is known, which has been constructed with a view toward avoiding programming errors. Thus, in Walters means are provided for counting the number of programming pulses received in synchronism with timing pulses produced within the pacemaker in order to assure that a number of pulses corresponding to the total desired number of pulses have been received. This function is performed by a code counting circuit 162 which is described at page 13, line 12 to 18 of the specification.

While this circuit will assure that reprogramming does not occur if the number of pulses received is less than the total desired number of pulses, there is no disclosure in Walters that the code counting circuit will repond in any way to the reception of a number of pulses greater than the total desired number. As a result, the reference circuit is still subject to faulty programming if a spurious signal interpreted as a program pulse should occur during the receiving interval.

## Disclosure of invention

The present invention relates to a programmable implantable body function control apparatus according to the pre-characterizing part of claim 1 which includes several advantages from the point of view of security for retaining the stored program and for only changing the stored program when desired. The apparatus provides a high degree of protection against the capability of noise changing the program.

According to the invention as described in the characterizing part of claim one there are provided program change enable means which permit the decoded signals to be entered into the program store to replace the program therein only when the number of bits received from the data signals equals the predetermined number of program bits of the program store and only when such predetermined number of program bits has been received by the program detection means within a given period of time.

The apparatus of the invention, by means of its program change enable means, thus includes circuitry which protects the program from being changed unless the correct number of data bits is transmitted to the implanted device within a predetermined period of time. If more or less program bits are received and decoded within the time allotted, then the program cannot be changed.

Preferably the data signals are pulse width modulated. When appropriately decoded, the data signals are preferably retained in a temporary store and then transferred into the program store when the conditions for actuating the program change enable means have been fulfilled.

In a typical embodiment of the invention, the body function control apparatus is a body tissue stimulator, in which case the control means comprises a stimulation pulse generator. Typically, the apparatus is an implantable programmable cardiac pacemaker of the demand or non-demand type where a change of program can be used to change the characteristics of the pacer stimulating pulses, for example the pulse rate, pulse width, pulse height.

Pulse width modulated programming provides a greater number of distinct programs per programming time period with a similar instrumentation as employed with known instrumentation based upon pulse counting or reed switch closure techniques.

Brief description of the drawings

A preferred embodiment of the invention is illustrated in the accompanying drawings, in which:

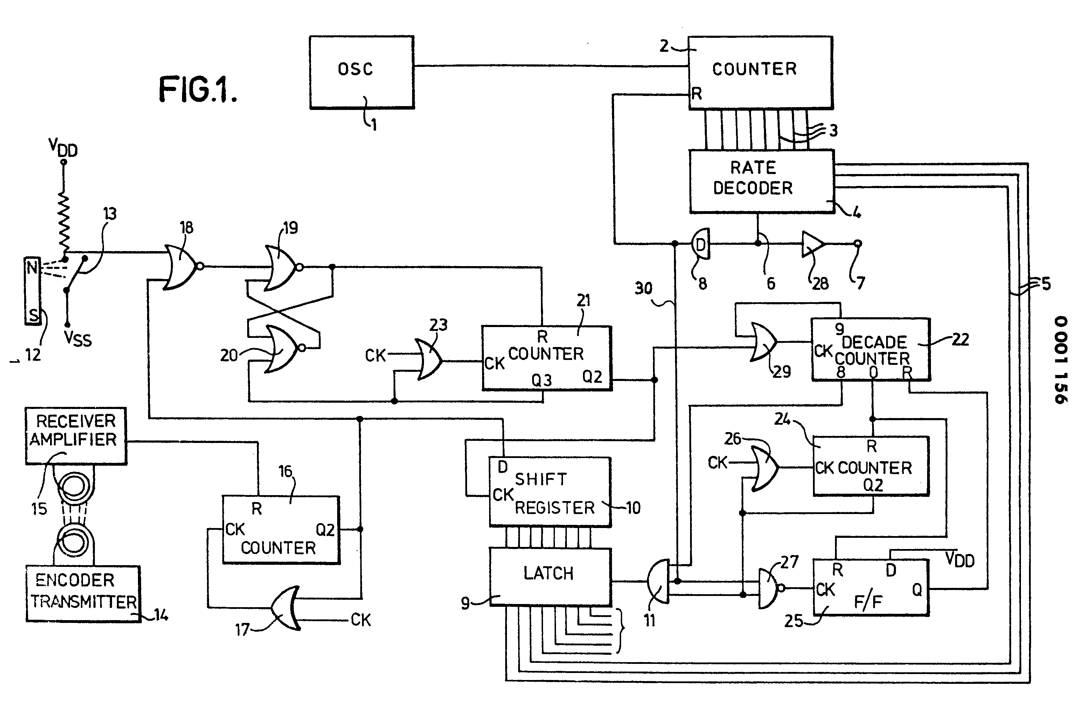

Figure 1 represents schematically an electrical circuit diagram of the body function control apparatus when in the form of an implantable, programmable cardiac pacemaker, and

Figure 2 represents a timing diagram for use in understanding Figure 1.

Best mode for carrying out the invention

Referring to the drawings, the pacemaker comprises an oscillator 1 which drives a ripple counter 2. The outputs of the various stages of the ripple counter are combined as is known in the art by means of logic gates (not shown) to provide eight output lines 3. The oscillator frequency and combination of ripple counter outputs are selected so that the eight output lines 3 provide, respectively, eight different body stimulation pulse frequencies (e.g. 40, 50, 60, 70, 80, 90, 100, and 110 pulses per minute). The eight output lines are supplied to a rate decoder 4 provided with three input control lines 5. By employing binary logic circuitry, the logic levels on the three lines 4 can be employed to select uniquely one from eight of the eight lines 3 and transmit the selected pulse frequency on line 6. Line 6 is connected via output amplifier 28 to an output terminal 7 and thence to a tissue stimulating pulse electrode disposed in or on the heart, and also to a delay unit 8 which, after an appropriate time, resets the counter 2, to enable the next appropriately timed pulse to be transmitted.

The bit values supplied on lines 5 to rate decoder 4 are obtained from an 8-bit latch 9 which stores a pacemaker program. As illustrated, three of the eight bits stored in latch 9 are employed to control the rate decoder 4 (i.e. the pulse rate) whereas the other five outputs from latch 9 are simply shown bracketed. These five outputs can be employed to control other pacemaker parameters as desired—e.g. pulse width, pulse height. The 8-bit latch values are set by corresponding values retained by a serial input/8-bit parallel output shift register 10. The shift register values are loaded into latch 9 upon recept of a store signal to the latter from an AND gate 11.

The manner in which the program stored by latch 9 is changed is dependent upon a magnet 12 being held close to the body so as to close reed switch 13, coincidentally with the detection and transmission to the shift register 10 of tone burst modulated data signals (for changing the pacemaker program) from an encoder/transmitter 14 also located outside the body. The coincidence of these two conditions provides a defense against noise being detected by the apparatus as data signals for changing the program, and is more fully described in the European Patent Applications Nos.

78 300 238.9 (Publication No. 0 000 985) and 78 300 240.5 (Publication No. 0 000 986). For the purposes of the presently-described embodiment "tone burst" modulation means pulse width modulation of a carrier frequency. The carrier is typically in the audio frequency range and with a sine wave modulator, but this need not be so and the invention is not so limited.

Encoder/transmitter 14 transmits tone burst modulated data signals to a receiver/amplifier 15, the output of which is employed to reset a counter 16. Counter 16 receives clock signals either from a system clock, advantageously 4 KHz., (e.g. from oscillator 1) or from its Q2 output stage, via an OR gate 17. The Q2 stage of counter 16 also drives the data input of shift register 10 and provides one input to NOR gate 18.

The second input to NOR gate 18 is derived from the normally high side of reed switch 13 which is connected between the electrical supply rails. NOR gate 18 output supplies one input to a pair of cross-coupled NOR gates 19, 20, with the output of NOR gate 19 being connected to the reset line of a ripple counter 21. Ripple counter 21 is such that the count pulses ripple successively through each of the counting stages—the lower stages do not remain high as the count proceeds to the higher stages. The Q2 output of ripple counter 21 is employed to clock shift register 10 and also a decade counter 22 via OR gate 29. The Q3 output of ripple counter 21 supplies one input to NOR gate 20 and to an OR gate 23. A second input to OR gate 23 is provided from a system clock, advantageously 250 Hz. (e.g. derived by subdividing clock pulses provided from oscillator 1) and the output of OR gate 23 is employed to clock ripple counter 21.

The zero stage of decade counter 22 is employed to reset a counter 24 and a flip-flop 25. The eighth stage of counter 22 provides one of three inputs to AND gate 11, and the ninth stage provides a return to OR gate 29 to lock out higher counts to counter 22. The rest line of the latter is driven by the Q output of the flip-flop 25.

Counter 24 is closed via an OR gate 26 from a system clock, advantageously 4 Hz. (e.g. derived by subdividing clock pulses provided from oscillator 1). Its Q2 output stage provides a return to OR gate 26 to provide a disable function on the latter. The Q2 output of counter 24 also provides a second of the three inputs to AND gate 11, and also an input to a NAND gate 27 which drives the clock input to flip-flop 25. The D-input line of flip-flop 25 is tied to the positive supply rail $V_{DD}$.

The third of the three inputs to AND gate 11 receives the reset pulse for ripple counter 2, this latter pluse also be supplied to another input of NAND gate 27.

The manner in which the program stored by latch 9 is decoded and then changed by the above circuit will first be described generally, and then in more detail.

The Q2 stage of counter 21, by clocking shift register 10, defines the time at which the signals received by counter 16 are analyzed. Counter 21 effectively analyzes the state of the Q2 stage of counter 16 at predefined instants of time and clocks either the 0 or 1 present at that stage into the shift register.

The shift register 10 only transfers its contents into the 8-bit latch 9, thereby changing the program, when the following three conditions are satisfied:—

1. When eight (and only eight) data bits have been clocked into shift register (10) (i.e. eight clock pulses to counter 22). This prevents the program being changed if less than or more than eight data bits have been received.

2. At a predetermined time after the receipt of the first data bit. This sets a maximum time limit within which all eight data bits must be received. The program will not therefore change if, say, only seven bits are received and then, after some longer period of time, a further data bit is received. If such a circumstance occurs, the circuit treats the received bits as spurious and will not change the program.

3. When an instant of time exists when counter 2 is being reset. This condition causes the program to change only in synchronism with the pacing pulses being transmitted. The program does not thus change at an instant of time at an intermediate stage in a count produced by counter 2, since this might have the effect of causing the pacemaker to issue two pacing pulses in rapid succession or to leave a gap where a pacing pulse ought to have been issued.

The above three conditions are met by clocking the 8-bit latch 9 from AND gate 11, the three inputs of which supply the three conditions above-mentioned. These condtions therefore have to coincide before AND gate 11 is enable so as to clock the shift register 10 contents into the 8-bit latch 9.

Decade counter 22 counts the number of data bits clocked into shift register 10 and only provides a high input to AND gate 11 after receipt of the eighth data bit (condition 1). Counter 24 provides a high input to AND gate 11 a predetermined time after its reset is removed (from the zero output from decade counter 22) (i.e. from the time the first data pulse is received—condition 2). The third high input to AND gate 11 occurs when counter 2 is reset (condition 3).

The operation of the decoding and storage of data bit pulses will now be described in greater detail. Assume that initially the output Q2 of counter 16 is high, and that the reset line to counter 21 also is high. With no magnet 12 adjacent reed switch 13 the latter will be open, providing a high input to NOR gate 18. Assume also that decade counter 22 is reset to zero, thus holding a reset on counter 24 and flip-flop 25. Assume now that it is desired to change the program stored in 8-bit latch 9. A magnet 12 is placed in proximity to the body adjacent the site of the implanted pacemaker, thus closing switch 13 and providing a low input to NOR gate 18. Simultaneously with the magnet emplacement, eight data bits for changing the program are transmitted from encoder/transmitter 14 for receipt and amplification by receiver/amplifier 15. The latter transmits to the reset terminal of counter 16 eight tone bursts of about 10 KHz frequency. A long tone burst is employed as a wide data "pulse" for storing a "0" in the 8-bit latch whereas a short tone burst is employed as a narrow data "pulse" for storing a "1".

The receipt of the first tone burst resets counter 16 causing NOR gate 18 output to provide a high output to NOR gate 19 which therefore provides a low output. This removes the reset on counter 21 which thus commences counting clock pulses. The first tone burst pulse continues to reset counter 16 but eventually counter 21 counts to its Q2 stage, thus clocking shift register 10. This has the effect, at this instant, of "analyzing" the output of the Q2 stage of counter 16 and inserting its value into the shift register. If the first tone burst has been a long burst, it is arranged that counter 16 will still be being reset by the tone burst when the analysis time provided by the Q2 output of counter 21 arrives. In such a circumstance a zero exists at the Q2 stage of counter 16 and is clocked into the shift register 10.

If the first tone burst is short, it is arranged that it will be terminated, so that counter 16 is no longer being reset thereby and has counted to its Q2 stage, by the time the shift register is clocked by counter 21. In such a circumstance, a "1" is clocked into the shift register. Counter 16 will hold at the Q2 stage since its output is supplied via OR gate 17 to the clock input to lock out any further clock pulses.

In this manner, a long tone burst will cause a "0" to be clocked into the shift register, and a short burst will cause "1" to be clocked into the shift register.

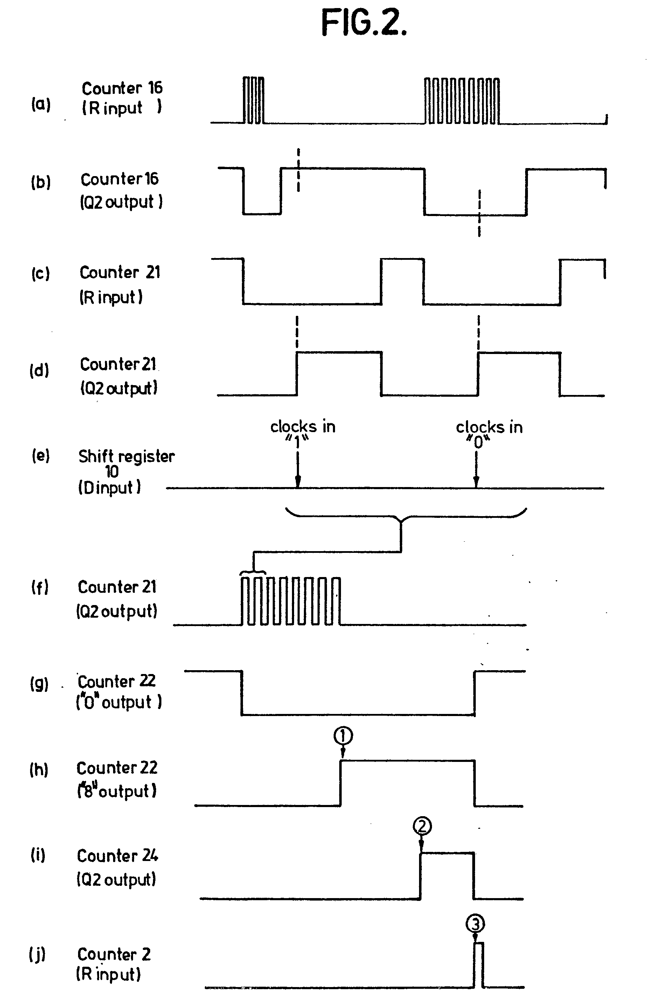

In Figure 2, the first tone burst illustrated is a short burst. The time at which the counter 21 analyzes the Q2 output of counter 16, for clocking the latter into the shift register, is shown by dashed vertical lines on pulse trains (b) and (d) in Figure 2. This short tone burst thus causes a "1" to be stored in the shift register. The second tone burst illustrated in Figure 2 is long, and this causes a "0" to be stored in the shift register.

Note also in Figure 2 that pulse trains (f) to (j) are compressed in time as compared to pulse trains (a) to (e). Pulse trains (d) and (f) are identical except that the former shows schematically only two pulses whereas the latter shows eight pulses.

The Q2 high output of counter 21 also provides a close pulse to decade counter 22 which thus counts the first data pulse now clocked in the shift register 10. After the arrival of two subsequent clock pulses to counter 21, the Q2 output goes low, and the Q3 output goes high to lock out further clock signals (via OR gate 23) until counter 21 is reset.

The clocking of decade counter 22 from zero to one removes the reset from counter 24, so that the latter commences counting clock pulses, and also removes the reset on flip-flop 25.

The locking of clock pulses provided by the Q2 stage on its counter 16, and the Q3 stage on is counter 21 causes a change in states of the NOR gates 18, 19, 20 which causes the NOR gate 19 output to revert high, thus resetting counter 21.

This situation remains until the next tone burst pulse arrives at the reset input to counter 16, when the above-described cycle is repeated causing another data bit to be clocked into shift register 10 and decade counter 22 to increment by "1". In the meantime, counter 24 continues to count clock pulses.

This cycle repeats until all eight tone bursts are received, analyzed and the appropriate bits clocked into shift register 10, which then retains a complete new program for the pacemaker.

Decade counter 22 has then reached a count of 8 to provide the first of the three input conditions to AND gate 11. Counter 24, still counting clock pulses, then "times out" by providing a high output on its Q2 stage, which locks out further clock pulses via OR gate 26 and provides the second of the three input conditions to AND gate 11. As soon thereafter that the pacing pulse-producing counter 2 is reset, the third of the three input conditions to AND gate 11 is satisfied, the output of which then goes high to load the 8-bits retained in the shift register 10 into the 8-bit latch 9, thereby changing with stored program.

Coinciding with the presence of the second and third of the input conditions for AND gate 11, NAND gate 27 provides a low output of short duration (duration of counter 2 reset). The low to high transition at NAND gate 27 clocks flip-flop 25, causing the latter to reset decade counter 22 to zero, the latter causing counter 24 and flip-flop 25 to reset. The circuit is then in the condition to receive another set of eight tone bursts to change the program at some future occasion.

It should be observed that if less than 8 tone bursts are received before conditions 2 and 3 are met, the first of the three input conditions of AND gate 11 will not be satisfied. This situation will also arise if more than 8 tone bursts are received before conditions 2 and 3 are met, since the ninth stage of decade counter 22 holds the clock input of the latter high to lock out any further clock pulses from counter 21. This holds counter 22 in the ninth stage until reset.

## Claims

1. A programmable, implantable body function control apparatus comprising: control means (1, 2, 3, 4, 6, 28, 7) for influencing a function of the body in accordance with a selected program, said control means including means (4) for changing the selected program; program storage means (9) for storing a predetermined number of bits representing the currently selected program and connected to said means for changing (4) for supplying the currently selected program to said control means; program detection means (10) connected to receive and temporarily store a predetermined number of bits representing a newly selected program, said program detection means (10) including output means connected to said program storage means (9) for supplying the bits received by said program detection means (10); signal conduction means (15) connected to receive bit signals provided by a source (14) outside the body and to supply a corresponding sequence of bits, representing such newly selected program, to said program detection means (10) and program change enable means (22, 24, 11) connected to said program storage means (9) for supplying a program change signal which causes the bits temporarily stored in said detection means (10) to be transferred into said program storage means (9) to replace the bits previously stored in said program storage means (9), characterized in that said program change enable means are composed of:

first counter means (22) connected for providing a count of the number of bits supplied to said program detection means (10), and for supplying a first enabling signal only when its count is equal to the predetermined number of bits;

timer means (24) connected for responding to the first bit supplied to said program detection means (10) and for supplying a second enabling signal at the end of a given period of time after response to such first bit; gating means (11) being connected to said first counter means (22) and said timer means (24) for supplying such program change signal to said program storage means (9) only when said first and second enabling signals are present in time coincidence, whereby the bits temporarily stored in said program detection means (10) can be transferred into said program storage means (9) only when precisely the predetermined number of bits has been supplied to said program detection means (10) within the given period of time.

2. An apparatus according to claim 1 for influencing a function of the body by providing stimulation pulses in accordance with said selected program, said means (4) for changing the selected program being provided in order to vary at least one parameter of the stimulation pulses, the selected program being represented by a sequence of bits in a pattern independent of that of the stimulation pulses, characterized in that said program change enable means further comprise synchronizing means (30) formed by coupling the output of said control means to said gating means (11) for supplying a third enabling signal in synchronism with each stimulation pulse provided by said control means, said third enabling signal ensuring that said program change signal is supplied to said program storage means (9) at a point of time which is in synchronism with a stimulation pulse provided by said control means.

3. An apparatus according to claim 1 or 2, characterized in that the program detection means (10) includes a temporary store in which the bits supplied by said conduction means (15) are retained, said program change enable means transferring the bits from said temporary store into said program storage means only when a program change signal is provided by switching means (12, 13).

4. An apparatus according to any of claims 1 to 3, characterized in that the bit signals received by said conduction means (15) are pulse width modulated.

5. An apparatus according to claim 4, characterized in that the bit signals received by said conduction means (15) are tone burst modulated.

6. An apparatus according to any of claims 1 to 5, characterized in that said program storage means (9) is a latch having capability for storage of said predetermined number of program bits.

7. An apparatus according to any of claims 1 to 6 when in the form of a body tissue stimulating apparatus and characterized in that said control means comprises a stimulation pulse generator.

8. An apparatus according to claim 7, characterized in that said pulse generator comprises an oscillator (1), and a second counter (2) driven by said oscillator providing at least two different pulse rates (3) said changing means including a rate decoder (4) whereby at least some of the program bits in said program storage means control said rate decoder to select one of the pulse rates provided by said second counter.

9. An apparatus according to claim 8, characterized in that the output pulse selected is arranged to reset via a delay unit (8) said second counter (2).

10. An apparatus according to claim 9, characterized in that said synchronizing means (30) is connected to provide said third enabling signal in time coincidence with resetting of said second counter (2).

11. An apparatus according to any of claims 7 to 10, characterized in that the body tissue stimulating apparatus is a cardiac pacemaker.

12. An apparatus according to claim 11, characterized in that cardiac pacemaker is a demand pacemaker.

13. In an implanted programmable cardiac pacer system having a selected plurality of programmably alterable functions respectively controllable from an external programmer, said programmer coupling to said pacer a plurality of input signal bits respectively corresponding to said alterable functions, a method for regulating said coupling while maintaining security of programming, comprising the steps of:

(a) detecting, at said implanted pacer, signal bits received at said programmer;

(b) maintaining an iterative count of signal bits so received; characterized in the further steps of:

(c) monitoring the accumulated time of said iterative count; and

(d) enabling a programming change in said pacer only if said iterative count reaches and does not exceed a predetermined count during a predetermined accumulated time.

14. A method according to claim 13 and further including the step of synchronizing said enabling step with the generation of pacing pulses by said pacer.

**Patentansprüche**

1. Eine programmierbare implantierbare Steuervorrichtung für Körperfunktionen, welche aufweist: Steuermittel (1, 2, 3, 4, 6, 25, 7), zur Beeinflussung einer Körperfunktion in Übereinstimmung mit einem ausgewählten Programm, wobei die Steuermittel (4) Mittel aufweisen, um das ausgewählte Programm zur verändern; Programmspeichermittel (9) zum Speichern einer vorbestimmten Anzahl von Bits welche das augenblicklich ausgewählte Program repräsentieren und mit den Mitteln zur Veränderung (4) verbunden sind, um das gegenwärtig ausgewählte Programm an die Steuermittel zu überführen; Programmerkennungsmittel (10), welche verbunden sind, um eine vorbestimmte Anzahl von Signalbits zu empfangen und zweitweise zu speichern, welche ein neu ausgewähltes Programm repräsentieren, wobei die Steuermittel (10) Ausgangsmittel enthalten, welche mit den Programmspeichermitteln (9) verbunden zind um die Bits von den Empfangsmitteln (10) zu liefern; Signalleitungsmittel (15), welche verbunden sind um Signal-Bits zu empfangen, die von einer Quelle (14) außerhalb des Körpers gelifert werden und um eine entsprechende Bitfolge absugeben, welche ein neu ausgewähltes Programm bilden, wobei die Programmerkennungsmittel (10) und Programmveränderungaktivierungsmittel (22, 24, 30, 11) mit dem Programmsteuermitteln (9) verbunden sind, um ein Programmwechselsignal abzugeben, welches veranlaßt, daß die in dem Erkennungsmitteln (10) zeitweise gespeicherten Bits in die Programmspeichermittel (9) gelangen, um die dort vorher gespeicherten Bits in dem Programmsteuermitteln (9) zu ersetzen, dadurch gekennzeichnet, daß die Programmsteuermittel enthalten:

erste Zählermittel (22), welche verbunden sind, um ein Zählergebnis der ersten Anzahl von Bits, welche an die Programmerkennungsmittel (10) geliefert wurden und um ein erstes Aktivierungssignal nur dann abzugeben, wenn der Zählerstand der vorbestimmten Anzahl von Bits entspricht;

Zeitgebermittel (24), welche auf das erste Bit ansprechen, welches an die Programmerkennungsmittel geliefert wird, um ein zweites Aktivierungssignal am Ende einer vorgegebenen Zeitdauer nach der Antwort auf das erste Bit abzugeben; Gattermittel (11), welche mit den ersten Zählmitteln (22) verbunden sind und Zeitgebermittel (24) zowie den Synchronisiermitteln (30), um ein derartiges Programmierungs-Wechselsignal an die Programmsteuermittel (9) nur dan abzugeben, wenn das erste und zweite Aktivierungssignal in zeitlicher Koinzidenz vorhanden sind, wobei die Bits, welche in dem Programmerkennungsmitteln (10) zeitweise gespeichert sind, in die Programmspeichermittel (9) nur dann übertragen werden können, wenn exakt die vorbestimmte Anzahl von Bits an die Programmerkennungsmittel (10) innerhalb der vorgegebenen Zeitdauer übertragen wurde.

2. Vorrichtung nach Anspruch 1, zur Abgabe von Stimulationsimpulsen entsprechend einem ausgewählten Programm, um mindestens einen Parameter der Stimulationsimpulse in einem Muster zu verändern, welches unabhängig von den Stimulationsimpulsen ist, dadurch gekennzeichnet, daß Synchronisationsmittel (30) vorgesehen sind, welche dadurch gebildet werden, daß das Ausgangssignal der Steuermittel an die Gattermittel (11) geleitet wird, um eine drittes Aktivierungssignal in Synchronität mit jedem Stimulationsimpuls, welcher von den Steuermitteln abgegeben wird, zu liefern, wobei die Gattermittel gleichzeitig mit den Synchronisationsmitteln (30) verbunden sind, um das Programmwechselsignal zu einem Zeitpunkt zu liefern, welcher synchron ist mit einem Stimulationsimpuls, welcher von den Steuermitteln abgegeben wird.

3. Vorrichtung nach Anspruch 1 oder 2, dadurch gekennzeichnet, daß die Programmerkennungsmittel (10) einen temporären Speicher enthalten in welchem die Bits, welche von Leitungsmitteln (5) abgegeben werden, wobei die Programmwechselaktivierungsmittel die Bits aus dem temporären Speicher in dem Programmspeicher nur dann übertragen, wenn ein Programmwechselsignal von Schaltmitteln (12, 13) abgegeben wird.

4. Vorrichtung nach einem der Ansprüche 1 bis 3, dadurch gekennzeichnet, daß die Bitsignale, welche von den Leitungsmitteln (15) empfangen werden, pulsdauermoduliert sind.

5. Vorrichtung nach Anspruch 4, dadurch gekennzeichnet, daß die Bitsignale, welche von den Leitungsmitteln (15) empfangen werden, burstsignalmoduliert sind.

6. Vorrichtung nach einem der Ansprüche 1 bis 5, dadurch gekennzeichnet, daß die Programmsteuermittel (9) aus einem Latch bestehen, welches eine vorbestimmte Anzahl von Programmbits aufnehmen kann.

7. Vorrichtung nach einem der Ansprüch 1 bis 6 in der Form eines Stimulators für Körpergewebe, dadurch gekennzeichnet, daß die Steuermittel einen Stimulationsimpulsgenerator enthalten.

8. Vorrichtung nach Anspruch 7, dadurch gekennzeichnet, daß der Impulsgenerator einen Oszillator (1) und einen zweiten Zähler (2) enthält, welcher von dem Oszillator mit zwei verschiedenen Impulsraten (3) angesteuert wird, wobei die Programmwechselmittel einen Ratendecoder (4) enthalten, mittels dessen mindestens einige der Programmbits in den Programmspeichermitteln den Ratendecoder zur Auswahl einer Pulsrate, welche von einem zweiten Zähler geliefert wird, ansteuern.

9. Vorrichtung nach Anspruch 8, dadurch gekennzeichnet, daß der ausgewählte Ausgangsimpuls herangezogen wird, um über eine Verzögerungseinheit (8) den zweiten Zähler (2) zurückzusetzen.

10. Vorrichtung nach Anspruch 9, dadurch gekennzeichnet, daß die Synchronisationsmittel derart verbunde sind, daß sie ein Aktivierungssignal in zeitliche Koinzidenz mit dem Zurücksetzen den zweiten Zählers (2) liefern.

11. Vorrichtung nach einem der Ansprüche 7 bis 10, dadurch gekennzeichnet, daß es sich bie der Stimulationsvorrichtung für Körpergewebe um einen Herzschrittmacher handelt.

12. Vorrichtung nach Anspruch 11, dadurch gekennzeichnet, daß es sich bei dem Herzschrittmacher um einen Demand-Schrittmacher handelt.

13. Bei einem implantierbaren, programmierbaren Herzschrittmachersystem mit einer Anzahl von programmierbar änderbaren Funktionen, welche jeweils von einem externen Programmiergerät zu beeinflussen sind, überträgt das Programmiergerät und den Schrittmacher eine Anzahl von Eingangssignalbits, welche jeweils verschiedenen veränderbaren Funktionen entsprechen, weist ein Verfahren zu Regulation der Kopplung unter Aufrechterhaltung der Programmiersicherheit die folgenden Schritte auf:

a) Erkennen, daß eine Anzahl von Signalbits vom implantierten Schrittmacher von einem Programmiergerät empfangen wurden;

b) Aufrechterhaltung der fortgesetzten Signalzählung der somit erhaltenen Bits gekennzeichnet durch die weiteren Schritte:

c) Überwachung der Gesamtheit der fortgesetzten Zählung und

d) Aktivierung des Programmwechsels in dem Schrittmacher nur dann, wenn die fortgesetzte Zählung innerhalb einer vorgegebenen Gesamtzeit einen vorgegebenen Zählerstand erreicht und nicht überschreitet.

14. Verfahren nach Anspruch 13, welches weiterhin den Schritt enthält, daß die Aktivierung mit der Abgabe eines Stimmulationsimpulses durch den Schrittmacher aktiviert ist.

## Revendications

1. Appareil de commande de fonction physiologique implantable programmable, comprenant: un moyen de commande (1, 2, 3, 4, 6, 28, 7) destinée à influencer une fonction du corps en fonction d'un programme choisi, ledit moyen de commande comportant un moyen (4) permettant de modifier le programme choisi; un moyen (9) d'emmagasignage de programme permettant d'emmagasiner un nombre prédéterminé de bits représentant le programme choisi en cours et connecté audit moyen de modification (4) afin de délivrer le programme choisi en cours audit moyen de commande; un moyen (10) de détection de programme connecté de façon à recevoir et temporairement emmagasiner un nombre prédéterminé de bits représentant un programme choisi nouveau, ledit moyen (10) de détection de programme comportant un moyen de sortie connecté audit moyen (9) d'emmagasinage de programme afin de délivrer les bits reçus par ledit moyen (10) de détection de programme; un moyen (15) de conduction de signal connecté de façon à recevoir des signaux de bits produits par une source (14) externe au corps et à délivrer une séquence correspondante de bits, représentant ce programme choisi nouveau, audit moyen (10) de détection de programme, et un moyen (22, 24, 11) de validation de modification de programme connecté audit moyen (9) d'emmagasignage de programme afin de délivrer un signal de modification de programme qui provoque le transfert des bits temporairement emmagasinés dans ledit moyen de détection (10) audit moyen (9) d'emmagasinage de programme pour remplacer les bits précédemment emmagasinés dans ledit moyen (9) d'emmagasinage de programme, caractérisé en ce que ledit moyen de validation de modification de programme est constitué de:

un premier moyen compteur (22) connecté de façon à produire une valeur de comptage du nombre de bits délivrés audit moyen (10) de détection de programme et à ne délivrer un premier signal de validation que lorsque sa valeur de comptage est égale au nombre prédéterminé de bits;

un moyen (24) de comptage de temps connecté de façon à répondre au premier bit délivré audit moyen (10) de détection de programme et à délivrer un deuxième signal de validation à la fin d'une durée donnée après la réponse à ce premier bit; un moyen (11) du type porte étant connecté audit premier moyen compteur (22) et audit moyen (24) de comptage de temps de façon à ne délivrer ledit signal de modification de programme audit moyen (9) d'emmagasinage de programme que lorsque ledit premier et ledit deuxième signal de validation sont présents en coïncidence de temps, si bien que les bits temporairement emmagasinés dans ledit moyen (10) de détection de programme ne peuvent être transférés dans ledit moyen (9) d'emmagasinage de programme que lorsque, de manière précise, le nombre prédéterminé de bits a été délivré audit moyen (10) de détection de programme dans les limites de la durée donnée.

2. Appareil selon la revendication 1, destiné à influencer une fonction du corps en fournissant des impulsions de stimulation selon ledit programme choisi, ledit moyen (4) permettant de modifier le programme choisi étant prévu de façon à faire varier au moins un paramètre des impulsions de stimulation, le programme choisi étant représenté par une séquence de bits d'un modèle indépendant de celui des impulsions de stimulation, caractérisé en ce que ledit moyen de validation de modification de programme comprend en outre un moyen de synchronisation (30) formé par couplage de la sortie dudit moyen de commande audit moyen (11) de type porte de façon à délivrer un troisième signal de validation en synchronisme avec chaque impulsion de stimulation produite par ledit moyen de commande, ledit troisième signal de validation assurant que ledit signal de modification de programme est délivré audit moyen (9) d'emmagasinage de programme à une instant quie est en synchronisme avec une impulsion de stimulation produit par ledit moyen de commande.

3. Appareil selon la revendication 1 ou 2, caractérisé en ce que le moyen de détection de programme (10) comporte une mémoire temporaire dans laquelle les bits délivrés par ledit moyen de conduction (15) sont conservés, ledit moyen de validation de modification de programme ne transférant les bits de ladite mémoire temporaire dans ledit moyen d'emmagasinage de programme que lorsque qu'un signal de modification de programme est produit par un moyen de commutation (12, 13).

4. Appareil selon l'une quelconque des revendications 1 à 3, caractérisé en ce que les signaux de bits reçus par ledit moyen de conduction (15) sont modulés en largeur d'impulsion.

5. Appareil selon la revendication 4, caractérisé en ce que les signaux reçus par ledit moyen de conduction (15) sont modulés en rafales d'audiofréquence.

6. Appareil selon l'une quelconque des revendications 1 à 5, caractérisé en ce que ledit moyen (9) d'emmagasinage de programme est une circuit de verrouillage possédant la capacité d'emmagasiner ledit nombre prédéterminé de bits de programme.

7. Appareil selon l'une quelconque des revendications 1 à 6, lorsqu'il se présente sous forme d'un appareil de stimulation de tissu du corps et caractérisé en ce que ledit moyen de commande comprend un générateur d'impulsions de stimulation.

8. Appareil selon la revendication 7, caractérisé en ce que le générateur d'impulsions comprend un oscillateur (1), et un deuxième compteur (2) excité par ledit oscillateur produisant au moins deux taux d'impulsion différents (3), ledit moyen de modification comportant un décodeur de taux (4), si bien qu'au moins une partie des bits de programme se trouvant dans ledit moyen d'emmagasinage de programme commande ledit décodeur de taux afin de sélectionner l'un des taux d'impulsions produits par ledit deuxième compteur.

9. Appareil selon la revendication 8, caractérisé en ce que l'impulsion de sortie choisie est destinée à repositionner, via une unité de retard (8), ledit deuxième compteur (2).

10. Appareil selon la revendication 9, caractérisé en ce que ledit moyen de synchronisation (30) est connecté de façon à produire ledit troisième signal de validation en coïncidence temporelle avec le repositionnement dudit deuxième compteur (2).

11. Appareil selon l'une quelconque des revendications 7 à 10, caractérisé en ce que l'appareil de stimulation de tissu du corps est un stimulateur cardiaque.

12. Appareil selon la revendication 11, caractérisé en ce que le stimulateur cardiaque est un stimulateur du type intervenant en cas de nécessité.

13. Dans un système stimulateur cardiaque programmable implanté possédant une pluralité choisie de fonctions modifiables par programme pouvant être respectivement commandées à partir d'un moyen de programmation externe, ledit moyen de programmation appliquant audit stimulateur plusieurs bits de signal d'entrée correspondant respectivement auxdites fonctions modifiables, un procédé permettant de réguler ladite application tout en maintenant la sécurité de programmation, comprenant les opérations suivantes:

(a) détecter, au niveau dudit stimulateur implanté, des bits de signal reçus au niveau dudit moyen de programmation;

(b) maintenir une valeur de comptage itérative des bits de signal ainsi reçus; caractérisé par les opérations supplémentaires suivantes:

(c) contrôler la durée accumulée de ladite valeur de comptage itérative; et

(d) ne valider une modification de programmation dans ledit stimulateur que si ladite valeur de comptage itérative atteint, sans la dépasser, une valeur de comptage prédéterminée pendant une durée accumulée prédéterminée.

14. Procééé selon la revendication 13 et comportant en outre l'opération consistant à synchroniser ladite opération de validation avec la production d'impulsions de stimulation par ledit stimulateur.

FIG.1.

OSC 1

COUNTER 2

3

RATE
DECODER 4

5

0 0 0 1 156

# FIG.2.

(a) Counter 16 (R input )

(b) Counter 16 (Q2 output )

(c) Counter 21 (R input )

(d) Counter 21 (Q2 output)

clocks in "1"     clocks in "0"

(e) Shift register 10 (D input )

(f) Counter 21 (Q2 output)

(g) Counter 22 ("0" output )

(h) Counter 22 ("8" output )

(i) Counter 24 (Q2 output)

(j) Counter 2 (R input)